# EUROPEAN PATENT SPECIFICATION

(11) **EP 2 568 810 B1**
(45) Date of publication and mention of the grant of the patent: **19.09.2018**
(21) Application number: 10851335.9
(22) Date of filing: 10.05.2010
(51) Int. Cl.: A01N 43/08, A61K 31/34, A61K 9/20

(54) **DARUNAVIR COMPOSITIONS**
DARUNAVIR-ZUSAMMENSETZUNGEN
COMPOSITIONS DE DARUNAVIR

(43) Date of publication of application: 20.03.2013
(73) Proprietor: Hetero Research Foundation, Hyderabad 500 018, Telangana (IN)
(72) Inventor: PARTHASARASHI REDDY, Bandi, Telangana (IN); KHADGAPATHI, Podili, Telangana (IN); KAMALAKAR REDDY, Goli, Telangana (IN)
(74) Representative: ZBM Patents ApS
(86) International application number: PCT/IN2010/000299
(87) International publication number: WO 2011/141921

(56) References cited:
- WO-A1-2010/086844
- WO-A2-2006/091529
- WO-A2-2009/013356
- US-A- 6 068 858
- US-A1- 2010 015 184
- VAN GYSEGHEM E ET AL: "Solid state characterization of the anti-HIV drug TMC114: Interconversion of amorphous TMC114, TMC114 ethanolate and hydrate", EUROPEAN JOURNAL OF PHARMACEUTICAL SCIENCES, ELSEVIER, AMSTERDAM, NL, vol. 38, no. 5, 8 December 2009 (2009-12-08), pages 489-497, XP026764329, ISSN: 0928-0987, DOI: 10.1016/J.EJPS.2009.09.013 [retrieved on 2009-09-24]

## Description

### Field of the invention

The present invention relates to an oral pharmaceutical composition of amorphous darunavir.

### Background of the Invention

Darunavir, also known as TMC-114 and UIC-94017, is a HIV-1 protease inhibitor. It selectively inhibits the cleavage of HIV encoded Gag-Pol polyproteins in infected cells, thereby preventing the formation of mature virus particles.

Darunavir is chemically [(3R,3aS,6aR)-2,3,3a,4,5,6a-Hexahydrofuro[5,4-b]furan-3-yl]N-[(2S,3R)-4-[(4-aminophenyl)sulfonyl-(2-methylpropyl)amino]-3-hydroxy-1-phenylbutan-2-yl]carbamate. Its empirical formula is C₂₇H₃₇N₃O₇S, and its molecular weight is 547.66. Darunavir has the following structural formula.

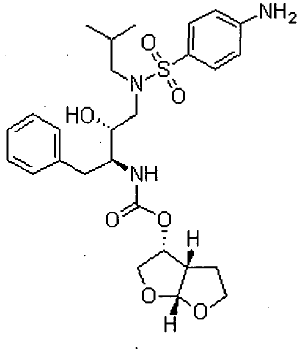

Darunavir is commercially available as tablets containing darunavir ethanolate under the trade name PREZISTA® in the United States, Europe and Canada.

The synthesis of darunavir and the manner in which it may be used to treat HIV infection are described in US patents 5843946, 6248775 and 6335460.

The present invention relates to an oral pharmaceutical composition comprising amorphous darunavir having d₉₀ particle size of about 150 µm to about 250 µm.

### Objective of the Invention

Accordingly, the main objective of the invention is to provide an oral pharmaceutical composition comprising amorphous darunavir having a d₉₀ particle size of about 150 µm to about 250 µm.

### Detailed description of the Invention

According to the present invention there is provided an oral pharmaceutical composition comprising amorphous darunavir having a d₉₀ particle size of 150 µm to 250 µm.

The preferable d₉₀ particle size of darunavir is in the range of 175 to 225 µm.

More preferably, the d₉₀ particle size of darunavir is in the range of 190 to 200 µm.

Preferably, the oral pharmaceutical composition is a solid oral dosage form.

The solid dosage form is in the form of tablet.

The tablet composition is optionally film coated.

The oral pharmaceutical composition of amorphous darunavir may be prepared by direct compression, wet granulation or roll compaction.

Preferably oral pharmaceutical composition of amorphous darunavir may be prepared by direct compression.

The oral pharmaceutical composition of the present invention may contain one or more additional excipients. These excipients may be selected from diluents, binders, disintegrants and lubricants.

Preferably, the diluent is selected from lactose, sucrose, glucose, mannitol, sorbitol, calcium carbonate, microcrystalline cellulose, Prosolv, magnesium stearate and mixtures thereof. More preferably, the diluent is Prosolv.

The preferable binder is selected from L-Hydroxy propyl cellulose, polyvinyl pyrrolidine, hydroxyl propyl methyl cellulose, hydroxyl ethyl cellulose and pre-gelatinized starch.

Preferably, the disintegrant is selected from croscarmellose sodium, crospovidone, sodium starch glycolate and low substituted hydroxyl propyl cellulose.

The more preferable disintegrant is selected from low substituted hydroxyl propyl cellulose and crospovidone.

Preferably, the lubricant is selected from sodium stearyl fumarate, magnesium stearate, zinc stearate, calcium stearate, stearic acid, talc, Glyceryl behenate and colloidal silicon dioxide.

More preferably, the lubricant is selected from magnesium stearate, zinc stearate, calcium stearate and colloidal silicon dioxide.

Prefearbly, the oral pharmaceutical composition comprises amorphous darunavir, prosolv, crospovidone, colloidal silicon dioxide and magnesium stearate.

The wet granulation process includes wet granulation of amorphous darunavir with the excipient (s), lubrication and followed by compression.

The compaction process includes compaction of amorphous darunavir with the excipient (s), lubrication and followed by compression.

The direct compression process includes blending amorphous darunavir with the excipient (s), lubrication and followed by compression.

The tablet composition is optionally film coated with opadry II orange.

The following examples further exemplify the invention and are not intended to limit the scope of the invention.

### Example 1:

| **Ingredients** | **Quantity /Unit (mg)** |
|---|---|
| Amorphous darunavir* | 600.00 |
| Prosolv | 583.75 |
| Crospovidone | 37.50 |
| Purified water | q.s |
| Colloidal silicon dioxide | 25.00 |
| Magnesium stearate | 3.75 |

| **Film coating** | |
|---|---|
| Opadry II orange | 25.00 |
| **Total Tablet weight** | **1275.00** |

| | |
|---|---|
| * Particle size distribution of amorphous darunavir: d₁₀-11 µm; d₅₀-50 µm d₉₀-195 µm | |

The process of the preparation involve following steps:
i). Blending of amorphous darunavir, prosolv, crospovidone dried if necessary
ii). Lubricating with colloidal silicon dioxide and magnesium stearate
iii). Compressing the lubricated blend of step (ii) into tablets.
iv). Coating the compressed tablets with opadry.

### Example 2:

| **Ingredients** | **Quantity /Unit (mg)** |
|---|---|
| Amorphous darunavir* | 600.00 |
| Prosolv | 596.25 |
| Crospovidone | 25.00 |
| Purified water | q.s |
| Colloidal silicon dioxide | 25.00 |
| Magnesium stearate | 3.75 |

| **Film coating** | |
|---|---|
| Opadry II orange | 25.00 |
| **Total Tablet weight** | **1275.00** |

| | |
|---|---|
| * Particle size distribution of amorphous darunavir: d₁₀-15 µm; d₅₀-60 µm d₉₀-210 µm | |

The process of the preparation involve following steps:
i). Granulation of amorphous darunavir, prosolv, crospovidone with a suitable sovent.
ii). Lubricating with colloidal silicon dioxide and magnesium stearate
iii). Compressing the lubricated blend of step (ii) into tablets.
iv). Coating the compressed tablets with opadry.

### Example 3:

| **Ingredients** | **Quantity /Unit (mg)** |
|---|---|
| Amorphous darunavir* | 600.00 |
| Prosolv | 608.75 |
| Crospovidone | 25.00 |
| Purified water | q.s |
| Colloidal silicon dioxide | 12.50 |
| Magnesium stearate | 3.75 |

| **Film coating** | |
|---|---|
| Opadry II orange | 25.00 |
| **Total Tablet weight** | **1275.00** |

| | |
|---|---|
| * Particle size distribution of amorphous darunavir: d₁₀-17 µm; d₅₀-65 µm d₉₀-213 µm | |

The process of the preparation involve following steps:
i). Compaction of Darunavir, prosolv, crospovidone with a suitable sovent.
ii). Lubricating with colloidal silicon dioxide and magnesium stearate
iii). Compressing the lubricated blend of step (ii) into tablets.
iv). Coating the compressed tablets with opadry.

### Example 4:

| **Ingredients** | **Quantity /Unit (mg)** |
|---|---|
| Amorphous darunavir* | 600.00 |
| Microcrystalline cellulose | 571.75 |
| Crospovidone | 37.50 |
| Purified water | q.s |
| Colloidal silicon dioxide | 37.00 |
| Magnesium stearate | 3.75 |

| **Film coating** | |
|---|---|
| Opadry II orange | 25.00 |
| **Total Tablet weight** | **1275.00** |

| | |
|---|---|
| * Particle size distribution of amorphous darunavir: d₁₀-8 µm; d₅₀-45 µm d₉₀-182 µm | |

The process of the preparation involve following steps:
i). Blending of amorphous darunavir, microcrystalline cellulose, crospovidone and dried if necessary
ii). Lubricating with colloidal silicon dioxide and magnesium stearate
iii). Compressing the lubricated blend of step (ii) into tablets.
iv). Coating the compressed tablets with opadry.

## Claims

1. An oral pharmaceutical composition in the form of tablets comprising darunavir having a d₉₀ particle size in the range of about 150 µm to 250 µm, wherein darunavir is in amorphous form.

2. The oral pharmaceutical composition according to claim 1, wherein the d₉₀ particle size is in the range of 150 to 225 µm.

3. The oral pharmaceutical composition according to claim 1, wherein the tablet is film coated.

4. The oral pharmaceutical composition according to claim 1, wherein the composition may contain one or more additional excipients.

5. The oral pharmaceutical composition according to claim 4, the excipients are selected from diluents, binders, disintegrants and lubricants.

6. The oral pharmaceutical composition according to claim 5, wherein the diluent is selected from lactose, sucrose, glucose, mannitol, sorbitol, calcium carbonate, microcrystalline cellulose, Prosolv, magnesium stearate and mixtures thereof.

7. The oral pharmaceutical composition according to claim 5, wherein the diluent is Prosolv.

8. The oral pharmaceutical composition according to claim 5, wherein the binder is selected from L-Hydroxy propyl cellulose, polyvinyl pyrrolidine, hydroxyl propyl methyl cellulose, hydroxyl ethyl cellulose and pre-gelatinized starch.

9. The oral pharmaceutical composition according to claim 5, wherein the disintegrant is selected from croscarmellose sodium, crospovidone, sodium starch glycolate and low substituted hydroxyl propyl cellulose.

10. The oral pharmaceutical composition according to claim 5, wherein the disintegrant is selected from low substituted hydroxyl propyl cellulose and crospovidone.

11. The oral pharmaceutical composition according to claim 5, wherein the lubricant is selected from sodium stearyl fumarate, magnesium stearate, zinc stearate, calcium stearate, stearic acid, talc, Glyceryl behenate and colloidal silicon dioxide.

12. The oral pharmaceutical composition according to claim 5, wherein the lubricant is selected from magnesium stearate, zinc stearate, calcium stearate and colloidal silicon dioxide.

13. The oral pharmaceutical composition according to claim 1, wherein the tablets of darunavir comprises amorphous darunavir, colloidal silicon dioxide, crospovidone, magnesium stearate and prosolv.

14. The oral pharmaceutical composition according to claim 1, wherein the tablets are prepared by direct compression.

## Patentansprüche

1. Eine orale pharmazeutische Zusammensetzung in Form von Tabletten umfassend Darunavir mit einer Partikelgröße d₉₀ im Bereich von etwa 150 µm bis 250 µm, wobei das Darunavir in amorpher Form ist.

2. Die orale pharmazeutische Zusammensetzung nach Anspruch 1, wobei die Partikelgröße d₉₀ im Bereich von 150 bis 225 µm liegt.

3. Die orale pharmazeutische Zusammensetzung nach Anspruch 1, wobei die Tablette filmbeschichtet ist.

4. Die orale pharmazeutische Zusammensetzung nach Anspruch 1, wobei die Zusammensetzung einen oder mehrere zusätzliche Hilfsstoffe umfassen kann.

5. Die orale pharmazeutische Zusammensetzung nach Anspruch 4, wobei die Hilfsstoffe aus Streckmitteln, Bindemitteln, Zerfallsbeschleunigern und Schmiermitteln ausgewählt sind.

6. Die orale pharmazeutische Zusammensetzung nach Anspruch 5, wobei das Streckmittel ausgewählt aus Lactose, Saccharose, Glucose, Mannitol, Sorbitol, Calciumcarbonat, mikrokristalliner Cellulose, Prosolv, Magnesiumstearat und Mischungen davon ausgewählt ist.

7. Die orale pharmazeutische Zusammensetzung nach Anspruch 5, wobei das Streckmittel Prosolv ist.

8. Die orale pharmazeutische Zusammensetzung nach Anspruch 5, wobei das Bindemittel ausgewählt aus L-Hydroxypropylcellulose, Polyvinylpyrrolidin, Hydroxylpropylmethylcellulose, Hydroxylethylcellulose und vorgelierter Stärke ist.

9. Die orale pharmazeutische Zusammensetzung nach Anspruch 5, wobei der Zerfallsbeschleuniger aus Croscarmellose-Natrium, Crospovidon, Natriumstärkeglykolat und niedrig substituierter Hydroxylpropylcellulose ausgewählt ist.

10. Die orale pharmazeutische Zusammensetzung nach Anspruch 5, wobei der Zerfallsbeschleuniger ausgewählt aus niedrig substituierter Hydroxylpropylcellulose und Crospovidon ist.

11. Die orale pharmazeutische Zusammensetzung nach Anspruch 5, wobei das Schmiermittel ausgewählt aus Natriumstearylfumarat, Magnesiumstearat, Zinkstearat, Calciumstearat, Stearinsäure, Talk, Glycerylbehenat und kolloidalem Siliziumdioxid ist.

12. Die orale pharmazeutische Zusammensetzung nach Anspruch 5, wobei das Schmiermittel ausgewählt aus Magnesiumstearat, Zinkstearat, Calciumstearat und kolloidalem Siliziumdioxid ist.

13. Die orale pharmazeutische Zusammensetzung nach Anspruch 1, wobei die Darunavir-Tabletten amorphes Darunavir, kolloidales Siliziumdioxid, Crospovidon, Magnesiumstearat und Prosolv umfassen.

14. Die orale pharmazeutische Zusammensetzung nach Anspruch 1, wobei die Tabletten durch Direktverpressung hergestellt werden.

## Revendications

1. Une composition pharmaceutique orale sous forme de comprimés comprenant du darunavir ayant une taille de particules d₉₀ dans l'intervalle d'environ 150 µm à 250 µm, dans laquelle le darunavir est sous forme amorphe.

2. La composition pharmaceutique orale selon la revendication 1, dans laquelle la taille de particules d₉₀ est dans l'intervalle de 150 à 225 µm.

3. La composition pharmaceutique orale selon la revendication 1, dans laquelle le comprimé est enrobé d'un film.

4. La composition pharmaceutique orale selon la revendication 1, dans laquelle la composition peut contenir un ou plusieurs excipients additionnels.

5. La composition pharmaceutique orale selon la revendication 4, dans laquelle les excipients sont choisis de diluants, de liants, d'agents de désagrégation et de lubrifiants.

6. La composition pharmaceutique orale selon la revendication 5, dans laquelle le diluant est choisi parmi la lactose, le saccharose, le glucose, le mannitol, le sorbitol, le carbonate de calcium, la cellulose microcristalline, le Prosolv, le stéarate de magnésium et des mélanges de ceux-ci.

7. La composition pharmaceutique orale selon la revendication 5, dans laquelle le diluant est le Prosolv.

8. La composition pharmaceutique orale selon la revendication 5, dans laquelle le liant est choisi parmi la L-hydroxypropylcellulose, la polyvinylpyrrolidine, l'hydroxylpropylméthylcellulose, l'hydroxyléthylcellulose et l'amidon prégélatinisé.

9. La composition pharmaceutique orale selon la revendication 5, dans laquelle l'agent de désagrégation est choisi parmi la croscarmellose de sodium, la crospovidone, le glycolate d'amidon de sodium et l'hydroxypropylcellulose faiblement substituée.

10. La composition pharmaceutique orale selon la revendication 5, dans laquelle l'agent de désagrégation est choisi parmi l'hydroxypropylcellulose faiblement substituée et la crospovidone.

11. La composition pharmaceutique orale selon la revendication 5, dans laquelle le lubrifiant est choisi parmi le stéarylfumarate de sodium, le stéarate de magnésium, le stéarate de zinc, le stéarate de calcium, l'acide stéarique, le talc, le béhénate de glycéryle et le dioxyde de silicium colloïdal.

12. La composition pharmaceutique orale selon la revendication 5, dans laquelle le lubrifiant est choisi parmi le stéarate de magnésium, le stéarate de zinc, le stéarate de calcium et le dioxyde de silicium colloïdal.

13. La composition pharmaceutique orale selon la revendication 1, dans laquelle les comprimés de darunavir comprennent du darunavir amorphe, du dioxyde de silicium colloïdal, de la crospovidone, du stéarate de magnésium et du prosolv.

14. La composition pharmaceutique orale selon la revendication 1, dans laquelle les comprimés sont préparés par compression directe.
